# EUROPÄISCHE PATENTANMELDUNG

(11) **EP 3 438 097 A1**
(43) Veröffentlichungstag der Anmeldung: **06.02.2019**
(21) Anmeldenummer: 17184005.1
(22) Anmeldetag: 31.07.2017
(51) Int. Cl.: C07D 265/22, C07D 249/18, C07F 9/6574

(54) **SYNTHESE VON 2-(4-(TRIFLUOROMETHYL)PHENYL)-4H-BENZO[D][1,3]OXAZIN-4-ON AUS 1-(4-TRIFLUOROMETHYLBENZOYL)-1H-BENZOTRIAZOL UNTER ZUGABE VON PALLADIUMCHLORID (PDCL2) UND BIPHEPHOS (6,6'-[(3,3'-DI-TERT-BUTYL-5,5'-DIMETHOXY-1,1'-BIPHENYL-2,2'-DIYL)BIS(OXY)]BIS(DIBENZO[D,F][1,3,2]DIOXAPHOSPHEPIN)) SOWIE ÄHNLICHE VERFAHREN**

(71) Anmelder: Evonik Degussa GmbH, 45128 Essen (DE)
(72) Erfinder: WU, Xiao-Feng, 18059 Rostock (DE); YIN, Zhiping, 18059 Rostock (DE); QI, Xinxin, 310018 Hangzhou (CN); BÖRNER, Armin, 18059 Rostock (DE); FRANKE, Robert, 45772 Marl (DE)

(57) **Zusammenfassung**

Synthese von 2-(4-(trifluoromethyl)phenyl)-4H-benzo[d][1,3]oxazin-4-on aus 1-(4-trifluoromethylbenzoyl)-1H-benzotriazol unter Zugabe von Palladiumchlorid (PdCl2) und BiPhePhos (6,6'-[(3,3'-Di-tert-butyl-5,5'-dimethoxy-1,1'-biphenyl-2,2'-diyl)bis(oxy)]bis(dibenzo[d,f][1,3,2]dioxaphosphepin)) und Zuführung von Kohlenmonoxid (CO) sowie ähnliche Verfahren zur Herstellung von 4H-Benzo[d][1,3]oxazin-4-on-Derivaten aus 1-Carbonyl-1H-Benzotriazol-Derivaten.

## Beschreibung

Die Erfindung betrifft ein Verfahren zur Synthese von Benzoxazinonen aus Triazolen.

Benzoxazinone bilden eine Klasse von anellierten Stickstoff-Heterocyclen, die aufgrund ihrer verschiedenen biologischen Aktivitäten bei der organischen Synthese von Interesse sind. In der Literatur sind verschiedene Methoden zu ihrer Herstellung entwickelt worden. Hierunterfindet die Cyclisierung von Anthranilsäure, N-Acylanthranilsäure oder Isotonsäureanhydrid die breiteste Akzeptanz. Alternative Methoden sind zwar bekannt, aber ihrer Anwendung in technischem Maßstab sind bisher durch die Verfügbarkeit von Substraten und die erforderlichen Reaktionsbedingungen Grenzen gesetzt.

Der Erfindung lag die Aufgabe zugrunde, ein Verfahren bereitzustellen, mit welchem Benzoxazinone aus Triazolen hergestellt werden können. Das Verfahren soll hierbei einen hohen Umsatz liefern.

Gelöst wird die Aufgabe durch eine Verfahren gemäß Anspruch 1.

Verfahren umfassend die Verfahrensschritte:
a) Zugabe eines Substrates der allgemeinen Formel (I): 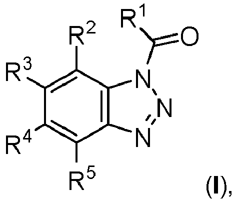 wobei
   R¹, R², R², R³, R⁴, R⁵ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl;
   wobei die genannten Alkylgruppen und Arylgruppen wie folgt substituiert sein können: substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen, -CH₂X, -CHX₂, -CX₃, -CH₂-CH₂X, -CH₂-CHX₂, -CH₂-CX₃, -CHX-CH₂X, -CHX-CHX₂, -CHX-CX₃, -CX₂-CH₂X, -CX₂-CHX₂, -CX₂-CX₃,
   mit X = Halogen;
b) Zugabe einer Verbindung, welche Pd umfasst, wobei das Pd in der Lage ist einen Komplex auszubilden;
c) Zugabe eines Liganden der allgemeinen Formel (**II**): 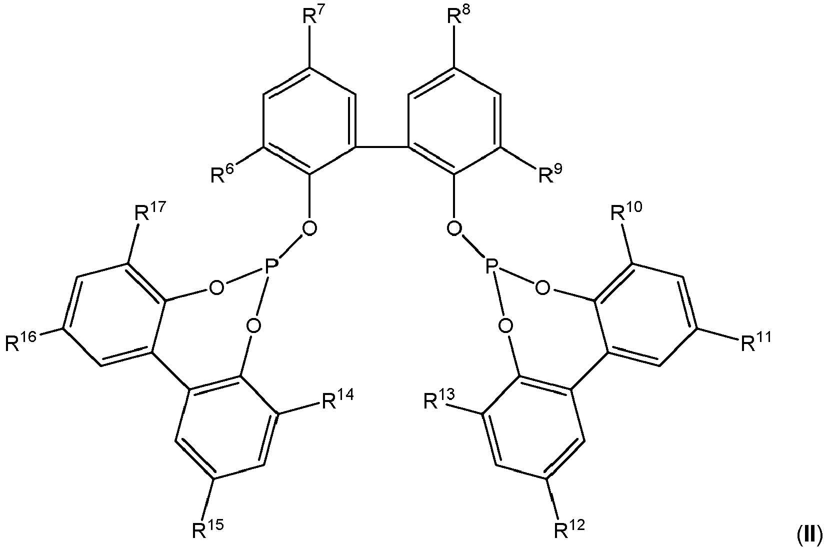
   wobei R⁶, R⁷, R⁸, R⁹, R¹⁰, R¹¹, R¹² R¹³, R¹⁴ R¹⁵ R¹⁶ R¹⁷ ausgewählt sind aus: -H, -(C₁-C₁₂)-Alkyl, -O-(C₁-C₁₂)-Alkyl,
   wobei die genannten Alkylgruppen wie folgt substituiert sein können:
      substituierte -(C₁-C₁₂)-Alkylgruppen, substituierte -O-(C₁-C₁₂)-Alkyl, können einen oder mehrere Substituenten aufweisen; die Substituenten sind unabhängig voneinander ausgewählt aus: -(C₁-C₁₂)-Alkyl, Halogen;
d) Zuführen von CO;
e) Erhitzen des Reaktionsgemisches, so dass die Verbindung (I) zur Verbindung (**III**) umgesetzt wird:

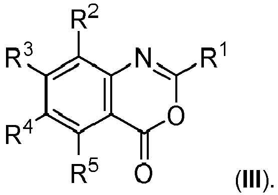

In einer Variante des Verfahrens steht X für F.

In einer Variante des Verfahrens ist die Verbindung in Verfahrensschritt b) ausgewählt aus: PdCl₂, PdBr₂, Pd(OAc)₂, Pd(dba)₂ (dba = Dibenzylidenaceton), PdCl₂(CHsCN)₂.

In einer Variante des Verfahrens ist die Verbindung in Verfahrensschritt b) PdCl₂.

In einer Variante des Verfahrens steht mindestens einer der Reste R⁶, R⁷, R⁸, R⁹, R¹⁰, R¹¹, R¹², R¹³, R¹⁴, R¹⁵ R¹⁶ R¹⁷ nicht für -H.

In einer Variante des Verfahrens steht mindestens einer der Reste R⁶, R⁷, R⁸, R⁹ nicht für -H.

In einer Variante des Verfahrens stehen R¹⁰, R¹¹, R¹², R¹³, R¹⁴, R¹⁵, R¹⁶, R¹⁷ jeweils für -H.

In einer Variante des Verfahrens sind R⁶, R⁷, R⁸, R⁹ ausgewählt aus: -(C₁-C₁₂)-Alkyl, -O-(C₁-C₁₂)-Alkyl.

In einer Variante des Verfahrens weist der Liganden (**II**) in Verfahrensschritt c) die Struktur (2) auf:

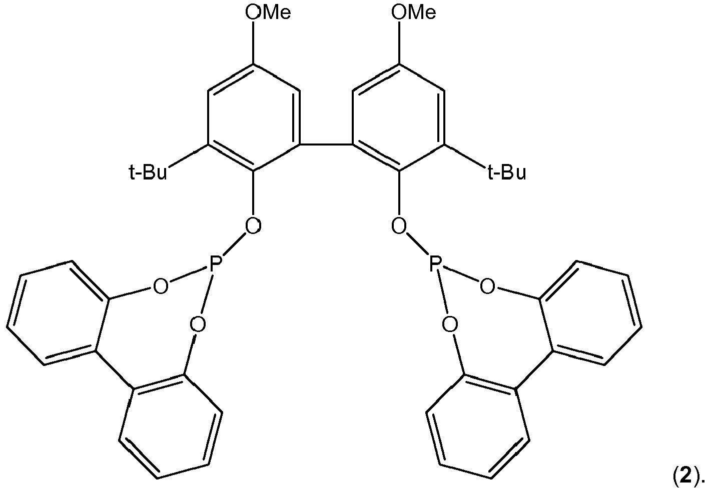

In einer Variante des Verfahrens umfasst dieses den zusätzlichen Verfahrensschritt f): f) Zugabe eines anorganischen Salzen ausgewählt aus: AgBF₄, AgF, Ag₂CO₃, Cu(OAc)₂.

In einer Variante des Verfahrens ist das anorganische Salz AgBF₄.

In einer Variante des Verfahrens umfasst dieses den zusätzlichen Verfahrensschritt g): g) Zugabe eines Lösungsmittels ausgewählt aus: CH₃CN, DMSO, DMF, tert-BuOH.

In einer Variante des Verfahrens ist das Lösungsmittel im Verfahrensschritt g) CH₃CN.

In einer Variante des Verfahrens erfolgt das Zuführen von CO im Verfahrensschritt d) so, dass die Reaktion bei einem CO-Druck im Bereich von 5 bar bis 40 bar abläuft.

In einer Variante des Verfahrens liegt der CO-Druck im Bereich von 10 bar bis 30 bar.

In einer Variante des Verfahrens erfolgt das Erhitzen im Verfahrensschritt e) auf eine Temperatur im Bereich von 80 °C bis 150 °C.

In einer Variante des Verfahrens erfolgt das Erhitzen im Verfahrensschritt e) auf eine Temperatur im Bereich von 100 °C bis 120 °C.

In einer Variante des Verfahrens weist das Substrates (I) die Formel (1) auf:

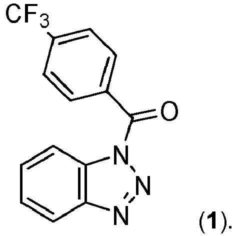

Im Folgenden wird die Erfindung anhand eines Ausführungsbeispieles näher erläutert. 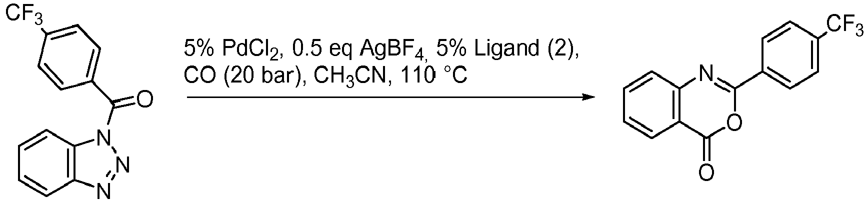 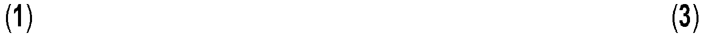 Ligand (2):

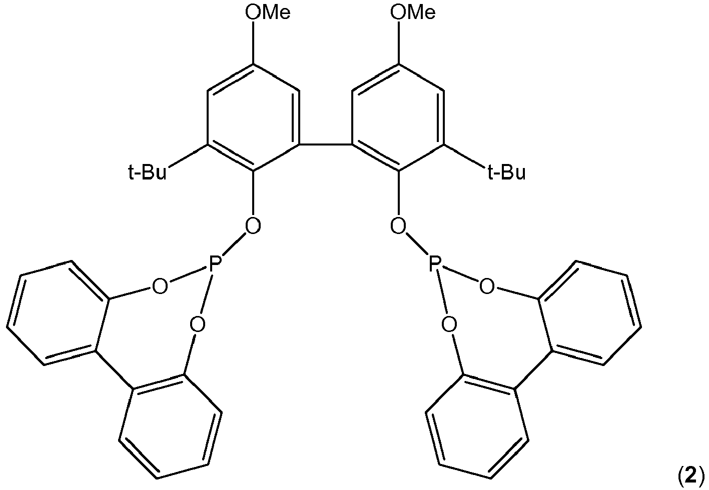

PdCl₂ (5 Mol-%), Ligand (2) (5 Mol-%), AgBF₄ (0,5 mmol) und Substrate (1) (1 mmol) wurden in ein Vial (Reaktionsvolumen 4 ml), das mit einem Septum, einer kleinen Kanüle und einem Rührstab ausgestattet war, überführt. Nach Spülen des Vial mit Argon wurde über eine Spritze MeCN (2 ml) in das Vial gegeben. Das Vial wurde dann unter Argonatmosphäre in einen Autoklaven (300 ml; Reihe 4560 von Parr Instruments) gestellt. Nach dreimaligem Spülen des Autoklaven mit CO wurde der Druck auf 20 bar eingestellt und die Reaktion 16 h bei 110 °C durchgeführt. Nach Abschluss der Reaktion wurde der Autoklav auf Raumtemperatur abgekühlt und vorsichtig entspannt. Die organische Lösung wurde unter Adsorption an Kieselgel im Vakuum eingedampft und durch Säulenchromatographie gereinigt. Es konnte ein Umsatz von 100 % erzielt werden.

¹H-NMR (300 MHz, Cdl₃): d 8.2 (d, 2H, J=8 Hz), 8.1 (dd, 1 H, *J*¹=8 Hz, *J*²=1 Hz), 7.6-7.8 (m, 1 H), 7.6 (d, 2H, J=8 Hz), 7.5-7.6 (m, 1 H), 7.3-7.5 (m, 1 H).
¹³C-NMR (75 MHz, CDCl₃): d 159, 155, 146, 137, 134 (q, J=33 Hz), 133, 129, 128.6, 128.4, 127, 126 (q, *J=4* Hz), 124 (q, J=273 Hz), 117.
GC-MS (EI, 70eV):m/z(%) = 291 (M+,100), 272 (10), 247 (65), 173 (40), 145 (50), 90 (10).

Wie das oben beschriebene Beispiel zeigt, wird die Aufgabe durch ein erfindungsgemäßes Verfahren gelöst.

## Patentansprüche

1. Verfahren umfassend die Verfahrensschritte:
a) Zugabe eines Substrates der allgemeinen Formel (I): 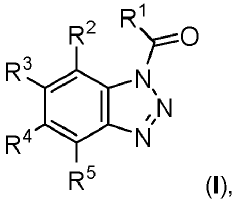 wobei
R¹, R², R², R³, R⁴, R⁵ ausgewählt sind aus: -H, -(C₁-C₁₂)-Alkyl, -O-(C₁-C₁₂)-Alkyl, -(C₆-C₂₀)-Aryl, -O-(C₆-C₂₀)-Aryl;
wobei die genannten Alkylgruppen und Arylgruppen wie folgt substituiert sein können:
substituierte -(C₁-C₁₂)-Alkylgruppen, substituierte -O-(C₁-C₁₂)-Alkyl, substituierte -(C₆-C₂₀)-Aryl, substituierte -O-(C₆-C₂₀)-Aryl können einen oder mehrere Substituenten aufweisen; die Substituenten sind unabhängig voneinander ausgewählt aus: -(C₁-C₁₂)-Alkyl, -O-(C₁-C₁₂)-Alkyl, Halogen, -CH₂X, -CHX₂, -CX₃, -CH₂-CH₂X, -CH₂-CHX₂, -CH₂-CX₃, -CHX-CH₂X, -CHX-CHX₂, -CHX-CX₃, -CX₂-CH₂X, -CX₂-CHX₂, -CX₂-CX₃, mit X = Halogen;
b) Zugabe einer Verbindung, welche Pd umfasst, wobei das Pd in der Lage ist einen Komplex auszubilden;
c) Zugabe eines Liganden der allgemeinen Formel (**II**): 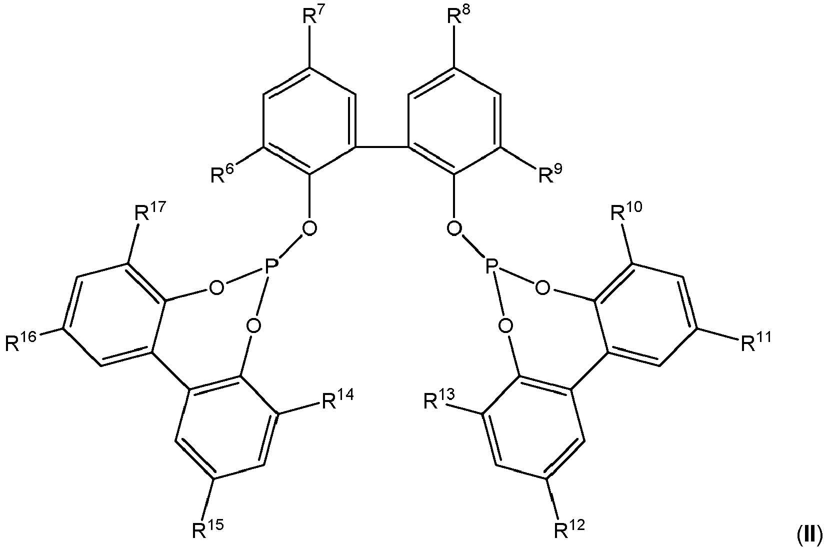
wobei R⁶, R⁷, R⁸, R⁹, R¹⁰, R¹¹, R¹² R¹³, R¹⁴, R¹⁵ R¹⁶ R¹⁷ ausgewählt sind aus: -H, -(C₁-C₁₂)-Alkyl, -O-(C₁-C₁₂)-Alkyl,
wobei die genannten Alkylgruppen wie folgt substituiert sein können:
substituierte -(C₁-C₁₂)-Alkylgruppen, substituierte -O-(C₁-C₁₂)-Alkyl, können einen oder mehrere Substituenten aufweisen; die Substituenten sind unabhängig voneinander ausgewählt aus: -(C₁-C₁₂)-Alkyl, Halogen;
d) Zuführen von CO;
e) Erhitzen des Reaktionsgemisches, so dass die Verbindung (I) zur Verbindung (**III**) umgesetzt wird:

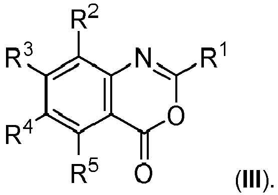

2. Verfahren nach Anspruch 1,
wobei die Verbindung in Verfahrensschritt b) ausgewählt ist aus:
PdCl₂, PdBr₂, Pd(OAc)₂, Pd(dba)₂ (dba = Dibenzylidenaceton), PdCl₂(CHsCN)₂.

3. Verfahren nach Anspruch 1 oder 2,
wobei mindestens einer der Reste R⁶, R⁷, R⁸, R⁹, R¹⁰, R¹¹, R¹², R¹³, R¹⁴, R¹⁵, R¹⁶, R¹⁷ nicht für -H steht.

4. Verfahren nach einem der Ansprüche 1 bis 3,
wobei mindestens einer der Reste R⁶, R⁷, R⁸, R⁹ nicht für -H steht.

5. Verfahren nach einem der Ansprüche 1 bis 4,
wobei R¹⁰, R¹¹, R¹² R¹³ , R¹⁴, R¹⁵ R¹⁶ R¹⁷ jeweils fur -H stehen.

6. Verfahren nach einem der Ansprüche 1 bis 5,
wobei R⁶, R⁷, R⁸, R⁹ ausgewählt sind aus: -(C₁-C₁₂)-Alkyl, -O-(C₁-C₁₂)-Alkyl.

7. Verfahren nach einem der Ansprüche 1 bis 6,
wobei der Liganden (**II**) in Verfahrensschritt c) die Struktur (2) aufweist:

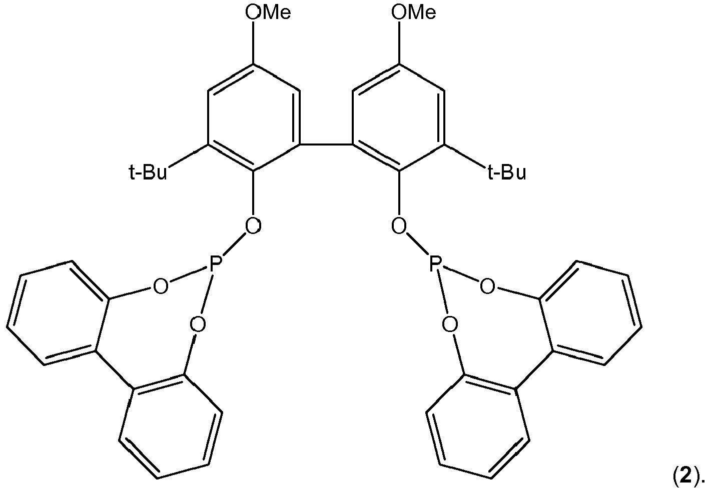

8. Verfahren nach einem der Ansprüche 1 bis 7,
umfassend den zusätzlichen Verfahrensschritt f):
f) Zugabe eines anorganischen Salzen ausgewählt aus: AgBF₄, AgF, Ag₂CO₃, Cu(OAc)₂.

9. Verfahren nach einem der Ansprüche 1 bis 8,
umfassend den zusätzlichen Verfahrensschritt g):
g) Zugabe eines Lösungsmittels ausgewählt aus: CH₃CN, DMSO, DMF, tert-BuOH.

10. Verfahren nach einem der Ansprüche 1 bis 9,
wobei das Zuführen von CO im Verfahrensschritt d) so erfolgt, dass die Reaktion bei einem CO-Druck im Bereich von 5 bar bis 40 bar abläuft.

11. Verfahren nach einem der Ansprüche 1 bis 10,
wobei das Erhitzen im Verfahrensschritt e) auf eine Temperatur im Bereich von 80 °C bis 150 °C erfolgt.

12. Verfahren nach einem der Ansprüche 1 bis 11,
wobei das Substrates (I) die Formel (1) aufweist:
